# EUROPEAN PATENT APPLICATION

(11) **EP 4 613 702 A1**
(43) Date of publication of application: **10.09.2025**
(21) Application number: 23885711.4
(22) Date of filing: 30.10.2023
(51) Int. Cl.: C01B 25/32, A61L 27/12, A61L 27/42

(54) **METHOD FOR MODIFYING CRYSTALLINE CALCIUM PHOSPHATE PARTICLES INTO AMORPHOUS CALCIUM PHOSPHATE BY WET GRINDING**

(30) Priority: 01.11.2022 US 202263421538 P; 25.02.2023 US 202363448282 P
(71) Applicant: Nagoya Institute Of Technology, Nagoya-shi, Aichi 466-8555 (JP); Orthorebirth Co., Ltd., Yokohama-shi, Kanagawa 224-0033 (JP)
(72) Inventor: KASUGA, Toshihiro, Nagoya-shi, Aichi 466-8555 (JP)
(74) Representative: Weihs, Bruno Konrad
(86) International application number: PCT/JP2023/039070
(87) International publication number: WO 2024/095956

(57) **Abstract**

A method of modifying crystalline calcium phosphate particles into amorphous calcium phosphate particles by wet-grinding using a bead mill apparatus without forming aggregates of the particles is proposed.

Powder of crystalline calcium phosphate particles and beads are put in a vessel of a bead mill apparatus such that ratio of apparent volume of the powder to the beads becomes 1:2-4, and acetone is added to the mixture of crystalline calcium phosphate powder and beads in the vessel such that weight ratio of the acetone to the mixture is 5-10 to 1, and stirring the mixture so that a slurry containing the calcium phosphate particles and beads in the acetone is prepared, and the bead mill apparatus is rotated at a predetermined rotation rate per minute, thereby grinding the powder of the crystalline calcium phosphate particles contained in the slurry without forming aggregates, and the crystalline calcium phosphate particles are partially amorphized.

## Description

### [Technical Field]

This invention relates to a method of modifying crystalline calcium phosphate particles into amorphous calcium phosphate by wet grinding using a bead mill apparatus, a method of loading metal or semi-metal ions onto the modified amorphous calcium phosphate particles, and a bone regeneration material that contains amorphous calcium phosphate particles produced by the method, and a method of producing such bone regeneration material.

### [Background Technology]

Recently, artificial bones in a cotton-wool like form made from biodegradable fibers containing calcium phosphate particles are used as a bone regeneration material. When the bone regeneration material is implanted in an affected area and comes into contact with a body fluid, calcium and phosphate ions contained in the biodegradable fibers are supplied at appropriate concentrations to stimulate bone-forming cells and promote their activity.

Hydroxyapatite (HAp) and beta-tricalcium phosphate (*β* -TCP) are used as calcium phosphate that are incorporated in a bone regeneration material. However, HAp is hardly dissolved when it comes into contact with a body fluid. *β* -TCP is dissolved by body fluid. However, because dissolution rate of *β* -TCP is small, it remains in a body for a long period of time after the bone regeneration material is implanted in the affected area. The *β* -TCP is gradually absorbed while gradually releasing calcium ions and phosphate ions.

It has been reported that supplying the affected area with metal or semi-metal ions, which are bio-functional ions, together with calcium and phosphate ions, further promotes bone formation (non-patent document 1). Magnesium is known to have an effect of promoting cell adhesion. It is known that biomaterials containing boron have a positive effect on the immune response, and that borate ions promote angiogenesis, which is one of the biological reactions closely related to bone repair. Silicon is known to stimulate osteoblasts. In addition, by loading silver onto biodegradable fibers, silver ions are released from the fibers to exert antibacterial properties.

It is possible to modify the crystal structure of ceramic particle such as calcium phosphate by applying impact energy. It has been reported that when *β* -TCP particles are wet-milled by applying impact energy using a ball mill, crystalline particles are amorphized by a mechanochemical reaction, and their solubility increases (non-patent document 2).

In order to use a mechanochemical reaction to modify crystalline calcium phosphate particles into amorphous calcium phosphate by wet grinding, it is necessary to apply a large impact energy to the particles. For this purpose, it is effective to use a planetary mill apparatus having high grinding capacity and apply a large impact energy to the particles using beads with a small media diameter. When wet grinding is conducted using a bead mill, the particles are ground to a finer state than the particles that are grounded using a ball mill, and the specific surface area of the particle increases dramatically. However, there is a problem that aggregation occurs in the powder of the fine particles. When aggregation occurs in the powder, it becomes difficult to uniformly disperse the particles in the spinning solution by adding the particles to the solvent and stirring them, and the aggregated particles settle near the bottom of the syringe used for spinning the dispersed solvent solution, blocking the area near the nozzle entrance through which the solution is passed for spinning, making spinning difficult.

Under the above circumstances, there has been a need for a method of improving or acquiring solubility in a body fluid by finely grinding calcium phosphate particles using a bead mill to modify the calcium phosphate particles into amorphous calcium phosphate particles without causing aggregation of the powder, and furthermore, a method of causing metal and semi-metal ions to be gradually released from the calcium phosphate particles.

### [Prior art document]

### [Non-patent document 1]

Non-patent document 1: Creation of a scaffold material for the sustained release of metal ions based on the activation mechanism of osteoblasts, MATERIA Vol. 59, No. 11 (2020) Akiko Obata, Toshihiro Kasuga

### [Non-patent document 2]

Non-patent document 2: Mechanical activation and cement formation of β -tricalcium phosphate Biomaterials Volume 24, Issue 23, October 2003 pages 4123-4131

### [Summary of the Invention]

### [Problems to be solved by the Invention]

In order to solve above problem, inventor of present invention studied the problem carefully and found that, as a result of grinding, surface of the crystalline calcium phosphate particles is broken down, and hydrogen is attached to the end of the P-0 group, forming a P-OH group. This OH group and water create a hydrogen bond, and this bond with other P-OH groups causes the particles to stick together and form aggregates. Based on this finding, the inventor of the present invention conceived that it would be possible to effectively prevent the occurrence of hydrogen bonding between ground particles by using a solvent that does not dissociate H⁺ and has no polarity in wet grinding.

Based on above idea, inventor of the present invention reached a method of modifying crystalline calcium phosphate particles into amorphous calcium phosphate by wet grinding using a bead mill apparatus, comprising:
putting a powder of crystalline calcium phosphate particles having a diameter of 1-5 *µ*m and beads having a media diameter of 0.015-2.0 mm in a vessel of a bead mill apparatus such that ratio of the apparent volume of the powder to the beads becomes 1:2-4, and
adding acetone to the mixture of crystalline calcium phosphate powder and beads in the vessel such that weight ratio of the acetone to the mixture is 5-10:1, and stirring the mixture so that a slurry containing the calcium phosphate particles and beads in the acetone is prepared, and
rotating the bead mill apparatus at a predetermined rotation rate per minute for a predetermined time, thereby grinding the powder of the crystalline calcium phosphate particles contained in the slurry to a particle size of 0.1-1.2 *µ*m without forming aggregates so that the crystalline calcium phosphate particles are partially amorphized by receiving impact from the beads and crystallinity of the calcium phosphate particles is reduced to 50-10%.

Preferably, the bead mill apparatus is a planetary type having a function of rotation and revolution.

Preferably, the crystalline calcium phosphate particles are *β* - TCP or HAp.

Preferably, the metal salt is silver phosphate.

Preferably, the semi-metallic salt is a silicate or a borate.

Preferably, the powder of the crystalline calcium phosphate particles and the powder of the metallic salt particles or the semi-metallic salt particles are mixed in a weight ratio of 0.99-0.9 to 0.01-0.1, and the mixture of the powders is put into the vessel of the planetary bead mill apparatus together with a predetermined amount of acetone and beads, and the planetary bead mill is rotated at a predetermined rate, and thereby the metal ions or semi-metal ions separated from the metal salt particles or semi-metal salt particles are coordinated with the phosphate ions of the amorphized calcium phosphate particles.

Inventor of present invention further developed calcium phosphate particles carrying metal or semi-metal ions in a gradually releasable manner, wherein
crystalline structure of the calcium phosphate particles is partially modified to an amorphous state by subjecting the particles to a bead milling,
metal or semi-metal ions are coordinated to the phosphate ions of amorphous part of the calcium phosphate particles so that the metal or semi-metal ions are carried on the calcium phosphate particles in a manner that the metal or semi-metal ions can be gradually released from the particles,
when the calcium phosphate particles carrying the metal or semi-metal ions are implanted in a body and come into contact with body fluids, the amorphous calcium phosphate particles are dissolved and the metal or semi-metal ions are gradually released together with calcium ions and phosphate ions.

Preferably, the bead milling uses a planetary-type apparatus having functions of rotation and revolution.

Preferably, the crystalline calcium phosphate particles are *β* - TCP or HAp.

Preferably, the metal salt is silver phosphate.

Preferably, the semi-metallic salt is a silicate or borate.

Inventor of present inventors further developed a method of producing a bone regeneration material comprising biodegradable fibers containing calcium phosphate particles that are modified to an amorphous state, the method comprising:
putting a powder of crystalline calcium phosphate particles having a diameter of 1-5 µm and beads having a media diameter of 0.015-2.0 mm in a vessel of a bead mill apparatus such that ratio of apparent volume of the powder to the beads becomes 1:2-4,
adding acetone to the mixture of crystalline calcium phosphate powder and beads in the vessel such that weight ratio of the acetone to the beads is 5-10 to 1, and stirring the mixture so that a slurry containing the calcium phosphate particles and beads in the acetone is prepared,
rotating the bead mill apparatus at a predetermined rotation rate per minute for a predetermined time, thereby grinding the powder of the crystalline calcium phosphate particles contained in the slurry to a particle size of 0.1-1.2 µm without forming aggregates so that the crystalline calcium phosphate particles are partially amorphized by receiving impact from the beads, and crystallinity of the calcium phosphate particles is reduced to 50-10%,
adding the powder of the amorphized calcium phosphate particles to a solvent together with biodegradable resin and stirring to produce a spinning solution in which the amorphized calcium phosphate particles are dispersed,
filling the spinning solution into a syringe of a spinning apparatus and extruding the spinning solution from a nozzle to spin biodegradable fibers, and
depositing the biodegradable fibers excluded from the nozzle in a collector and collecting the biodegradable fibers from the collector.

Preferably, the bead mill apparatus is a planetary type having a function of rotation and revolution.

Preferably, the calcium phosphate particles are *β* -TCP or HAp.

Preferably, metal or semi-metal ions are combined with the calcium phosphate particles that are modified to amorphous phase.

The inventor of present invention further developed a bone regeneration material comprising a biodegradable fiber containing calcium phosphate particles that are modified to amorphous state, the bone regeneration material is produced by the method comprising:
putting a powder of crystalline calcium phosphate particles having a diameter of 1-5 µm and beads having a media diameter of 0.015-2.0 mm in a vessel of a bead mill apparatus such that ratio of apparent volume of the powder to the beads becomes 1:2-4,
adding acetone to the mixture of crystalline calcium phosphate powder and beads in the vessel such that weight ratio of the acetone to the mixture is 5-10 to 1, and stirring the mixture so that a slurry containing the calcium phosphate particles and beads in the acetone is prepared,
rotating the bead mill apparatus at a predetermined rotation rate per minute for a predetermined time, thereby grinding the powder of the crystalline calcium phosphate particles contained in the slurry to a particle size of 0.1-1.2 µm without forming aggregates so that the crystalline calcium phosphate particles are partially amorphized by receiving impact from the beads, and crystallinity of the calcium phosphate particles is reduced to 50- 10%,
adding the powder of the amorphized calcium phosphate particles to a solvent together with biodegradable resin and stirring to produce a spinning solution in which the amorphized calcium phosphate particles are dispersed,
filling the spinning solution into a syringe of a spinning apparatus and extruding the spinning solution from a nozzle to spin biodegradable fibers,
depositing the biodegradable fibers excluded from the nozzle in a collector and collecting the biodegradable fibers from the collector.

Preferably, the bead mill apparatus is a planetary type having a function of rotation and revolution.

Preferably, the calcium phosphate particles are *β* -TCP or HAp.

Preferably, the calcium phosphate particles modified to the amorphous state are loaded with metal or semi-metal ions that can be released gradually.

### [Effect of the Invention]

By amorphizing the crystalline calcium phosphate particles through bead milling, the calcium phosphate, which does not dissolve in aqueous solution, acquires solubility, and the solubility of *β* - TCP, which has a slow dissolution rate, can be increased.

By implanting calcium phosphate particles that are made finer and amorphized by the bead milling process described in the present invention in a living body, calcium and phosphate ions are supplied from the calcium phosphate particles early after implantation.

By subjecting the calcium phosphate particles to bead milling, the particles are made finer and amorphous, and the phenomenon of the particles' surfaces becoming electrically charged and metal and semimetal ions being carried by the particles, and the particles colliding with each other and becoming integrated, occurs one after another, making it possible to contain metal and semimetal ions in the particles. By implanting calcium phosphate particles containing metal or semi-metal ions into the body, it is possible to gradually release metal or semi-metal ions together with calcium ions and phosphate ions from the calcium phosphate particles after implantation.

The calcium phosphate particles that are made fine and amorphous by the bead mill treatment of this invention are not aggregated, so it is possible to easily disperse and contain the fine powder in the spinning solution by putting the powder into a solvent together with biodegradable resin and stirring it. Using the spinning solution prepared in this way, it is possible to produce bone regeneration materials in which calcium phosphate particles are uniformly dispersed and contained in biodegradable fibers by the electrospinning method or the wet spinning method.

By preparing a spinning solution containing calcium phosphate particles that have been made fine and amorphous and then impregnated with metal or semi-metal ions, and then spinning it using the electrospinning method or wet spinning method, it is possible to produce an excellent bone regeneration material that has both high osteogenic potential and antibacterial or angiogenic potential.

### [Brief Explanation of the Drawings]

Figure 1: Figure 1 (A) shows a photograph of the external appearance of the planetary bead mill apparatus used in an embodiment of present invention, and Figure 1 (B) shows a schematic diagram of the rotation and revolution of the same apparatus.
Figure 2: Figure 2 (A) shows a scanning electron microscope (SEM) image of *β* -TCP particles that underwent bead milling for 120 minutes, and Figure 2 (B) shows the particle size distribution calculated from the image. In the figures, the bar graph shows the number of particles, and the line graph shows the cumulative number of particles.
Figure 3: Figure 3 shows the relationship between processing time of bead milling and the particle size of *β* -TCP, and indicates the particle size at the 10%, 50%, and 90% positions on the cumulative particle size distribution curve.
Figure 4: Figure 4 shows the relationship between processing time of bead milling and specific surface area of the resulting powder. Milling time 0 in the figure indicates the powder before processing.

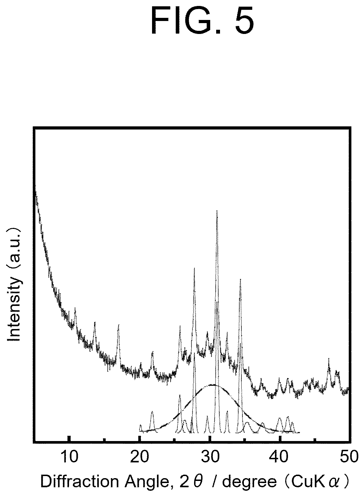

Figure 5: Figure 5 shows the X-ray diffraction pattern of *β* -TCP (Taihei Chemical Industry Co., Ltd. product, grinded): acetone: 1 mm zirconia beads at a weight ratio of 1:7.5:22.5, for a total weight of 55.8 g, placed in a 45 mL zirconia vessel and milled by planetary rotation at a speed of 400 rpm for 120 minutes in that the milling was carried out by planetary rotation at a speed of 800 rpm for 120 minutes. Spectrum of CuK α 2 *θ* = 20-43° was separated into peaks using a Gaussian function, and the crystal phase peaks and amorphous phase peaks were separated. The dashed line in the figure is the peak separated as the amorphous phase.
Figure 6: Figure 6 shows the relationship between the bead milling time and the amorphous phase content in the resulting powder. Milling time 0 in the figure refers to the powder before processing. 31P Solid-state nuclear magnetic resonance MAS-NMR spectrum peaks were separated into crystalline and amorphous phases using a Gaussian function, and the amorphous phase content ratio is plotted.
Figure 7: Figure 7 shows the results of determining the amount of calcium and phosphate ions that are eluted after 30 mg of powder was soaked in 20 mL of 37° C tris-HCl buffer solution (pH 7.40) for 24 h using inductively coupled plasma atomic emission spectrometry (ICP-AES).
Figure 8: Figure 8 shows the SEM photograph of the product obtained by milling *β* -TCP (Taihei Chemical Industry Co., Ltd. product, ground): silver phosphate (FUJIFILM Wako Pure Chemical Corporation product, Ag3P04): acetone: 1mm Φ zirconia beads at a weight ratio of 0.99:0.01:7.5:22.5, with a total weight of 55.8g. These are placed in a 45 mL zirconia vessel and milled by planetary rotation at a speed of 400 rpm for orbital rotation and 800 rpm for rotation around the axis.

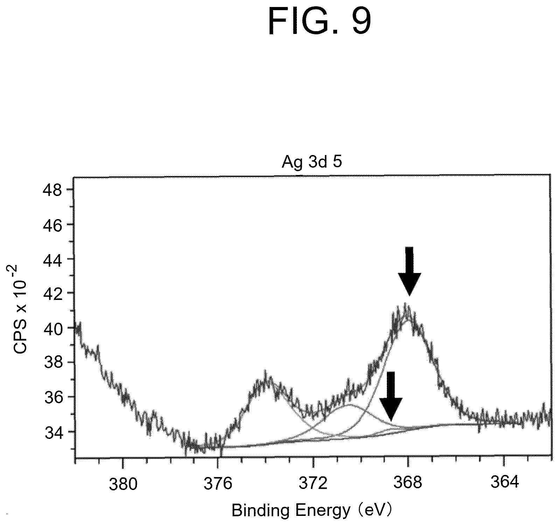

Figure 9: Figure 9 shows the results of milling *β* -TCP (ground product produced by Taihei Chemical Industry Co., Ltd.): silver phosphate (Ag3P04 manufactured by FUJIFILM Wako Pure Chemical Corporation): acetone: 1 mm diameter zirconia beads at a weight ratio of 0.99:0.01:7.5:22.5, for a total weight of 55.8 g. These are placed in a 45 mL zirconia vessel and milled using planetary rotation at a speed of 400 rpm for orbital rotation and 800 rpm for rotation around the axis. The Ag 3d spectrum obtained from X-ray photoelectron spectroscopy measurement is shown. The peaks were separated using a Gaussian function, and the peak intensities of the Ag ion appearing at 368 eV and the metallic Ag appearing at 369 eV were compared. The results showed that almost all of the silver was introduced as an ion.

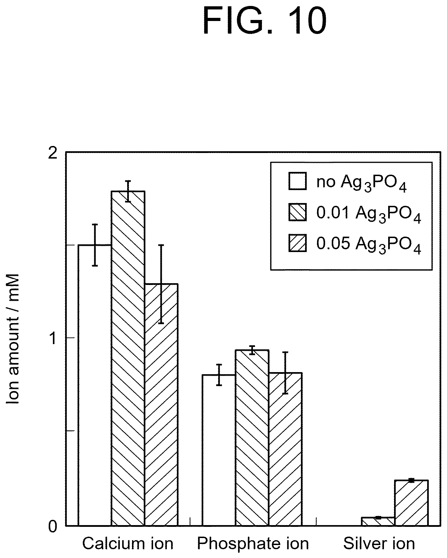

Figure 10: Figure 10 shows the results of milling *β* -TCP (Taihei Chemical Industry Co., Ltd.), silver phosphate (Ag3P04, FUJIFILM Wako Pure Chemical Corporation), acetone, and 1 mm diameter zirconia beads in a weight ratio of (1-x):x:7.5:22.5 (x=0, 0.01, 0.05), for a total weight of 55.8 g. These were placed in a 45 mL zirconia vessel and milled by planetary rotation at a speed of 400 rpm for orbital rotation and 800 rpm for rotation around the axis, and the amount of calcium, phosphate, and silver ions eluted by immersing 80 mg of the milled material in 40 mL of HEPES buffer solution at 37° C for 24 h was measured by ICP-AES.
Figure 11: Figure 11 shows a transmission electron microscope (TEM) image and electron diffraction image of *β* -TCP particles that have been bead-milled for 120 minutes.
Figure 12: Figure 12 shows the XRD pattern of *β* -TCP particles that have been bead-milled for 120 minutes and then immersed in a 37° C tris-HCl buffer solution for up to 72 hours.
Figure 13: Figure 13 shows the XRD pattern of *β* -TCP (Taihei Chemical Industry Co., Ltd. product, ground): acetone or water: 1 mm zirconia beads, with a weight ratio of 1:7.5:22.5, for a total weight of 55.8 g, placed in a 45 mL zirconia vessel, milled by planetary rotation at a speed of 400 rpm for 120 minutes.
Figure 14: Figure 14 shows SEM images of particles obtained by mixing and milling *β* -TCP powder and B₂O₃ powder using the bead milling process of the invention. Those that do not contain B₂O₃ are described as OB-CP, and those that contain 10-30% B₂O₃ are described as 10 ~ 30B-CP. The particle size is approximately 0.1 *µ*m-1*µ*m, and the shape is shown to vary.
Figure 15: Figures 15 (A), (B) and (C) show EDS mapping images of the particles in Figure 14. Figure 15 (A) shows the particles with 10% B₂O₃ mixed, Figure 15 (B) shows the particles with 20% B₂O₃ mixed, and Figure 15 (C) shows the particles with 30% B₂O₃ mixed.
Figure 16: Figure 16 shows the XRD patterns of the powders of the particles in Figure 14. Those that do not contain B₂O₃ are described as OB-CP, and those that contain 10 ~ 30% B₂O₃ are described as 10 ~ 30B-CP.
Figure 17: Figure 17 shows the 11B MAS-NMR spectrum (B₂O₃ mixed at 10-30%) of the powder of the particles in Figure 14. Q indicates boron with four oxygen coordination, T2 indicates boron with three oxygen coordination, where two of the oxygens are bridging oxygens, and T3 indicates boron with three oxygen coordination, where all of the oxygens are bridging oxygens.
Figure 18: Figure 18 shows the 31P MAS-NMR spectrum of the powdered particles in Figure 14 (those that do not contain B₂O₃ are described as OB-CP, and those that contain 10-30% B₂O₃ are described as 10-30B-CP).
Figure 19: Figures 19 (A), (B), and (C) show the amount of ion elution after immersion of the particles in Figure 14 in tris-HCl buffer solution. In the figures, OB-CP indicates the particles without B₂O₃, and 10-30B-CP indicates the particles with 10-30% B₂O₃ mixed in.
Figure 20: Figure 20 shows the XRD pattern of the powder obtained by immersing the particles in Figure 14 in a tris-HCl buffer solution for 120 minutes (OB-CP, B₂O₃ mixed with 10-30%: 10-30B-CP).
Figure 21: Figure 21 shows the XRD pattern of the particles obtained by mixing and milling *β* -TCP powder and SiO2 powder using the bead milling process of this invention. For comparison, the XRD patterns of *β* -TCP powder and *β* -TCP powder that has been bead milled are also shown.
Figure 22: Figure 22 (A) shows an elemental mapping diagram obtained by energy dispersive X-ray spectroscopy (EDS) of particles obtained by mixing and milling *β* -TCP powder and SiO₂ powder at a weight ratio of 95:5 using the bead milling process of this invention, and Figure 22 (B) shows the spectra of points 1 to 5.
Figure 23: Figure 23 shows the ion concentrations at various times when 50 mg of *β* -TCP powder mixed with 5% SiO2 was immersed in 20 mL of tris-HCl buffer solution at 37° C for a specified time.
Figure 24: Figure 24 shows the XRD pattern of the powder after immersion in the tris-HCl buffer solution in Figure 23.
Figure 25: Figure 25 (A) shows the amount of calcium ions in the solution after immersing biodegradable fiber produced by mixing m-TCP and PDLLGA in a weight ratio of 70-30 (Fiber A), biodegradable fiber produced by mixing *β* -TCP, m-TCP and PDLLGA in a weight ratio of 35:35:30 (Fiber B), and biodegradable fiber produced by mixing *β* -TCP and PDLLGA in a weight ratio of 70:30 (Fiber C) in a pH 7.40 tris-HCl buffer solution for a specified time respectively,

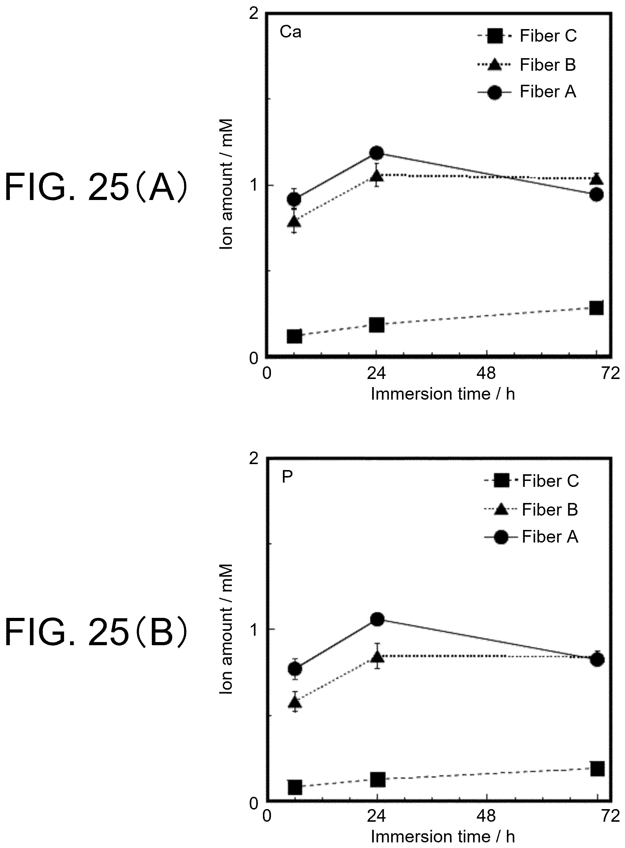

Figure 25 (B) shows the amount of phosphate ions in the solution after immersing biodegradable fiber produced by mixing m-TCP and PDLLGA in a weight ratio of 70-30 (Fiber A), biodegradable fiber produced by mixing *β* -TCP, m-TCP and PDLLGA in a weight ratio of 35:35:30 (Fiber B), and biodegradable fiber produced by mixing *β* -TCP and PDLLGA in a weight ratio of 70:30 (Fiber C) in a pH 7.40 tris-HCl buffer solution for a specified time respectively, Figure 25 (B) shows the amount of calcium ions in the solution after the beads-milled calcium phosphate particles (m-TCP) and the poly(D,L-lactic acid-co-gluconic acid) copolymer (PDLLA) were mixed in a weight ratio of 70:30 to make a biodegradable fiber (Fiber A), a fiber with a weight ratio of 35:35: 30 (Fiber B), and a fiber with a weight ratio of 70:30 for *β* - TCP:PDLLA (Fiber C) were immersed in a pH 7.40 tris-HCl buffer solution for a specified time, and the amount of phosphate ions in the solution after immersion is shown.
Figure 26: Figure 26 shows the XRD pattern of the fiber in Figure 25, which was immersed in a tris-HCl buffer solution for 72 h.

### <Embodiment of invention>

Hereinafter, preferred embodiments of present invention are explained with reference to the drawings.

### [Definition of Terms]

### <Bead milling>

In the present invention "bead milling" refers to a processing that uses small beads as the grinding media in a milling apparatus used for mechanochemical processing. The diameter of the beads used as the grinding media in bead milling is 0.015 to 0.5 mm, whereas the diameter of the balls used as the grinding media in ordinary ball milling is 10 to 50 mm. By using beads with a small diameter as the grinding media, it is possible to impart 100 to 500 times the energy to the calcium phosphate particles as compared to using balls with a diameter of 10 to 50 mm.

### <Ball mill apparatus>

In the present invention, the term "ball mill apparatus" refers to a grinding machine used to apply a mechanochemical treatment to particles, and which uses balls with a diameter of 10 to 50 mm as grinding media. A planetary ball mill apparatus can grind particles with higher efficiency by applying impacts to particles placed in a vessel (pot) by alternately repeating rotation and revolution.

### <Bead mill apparatus>

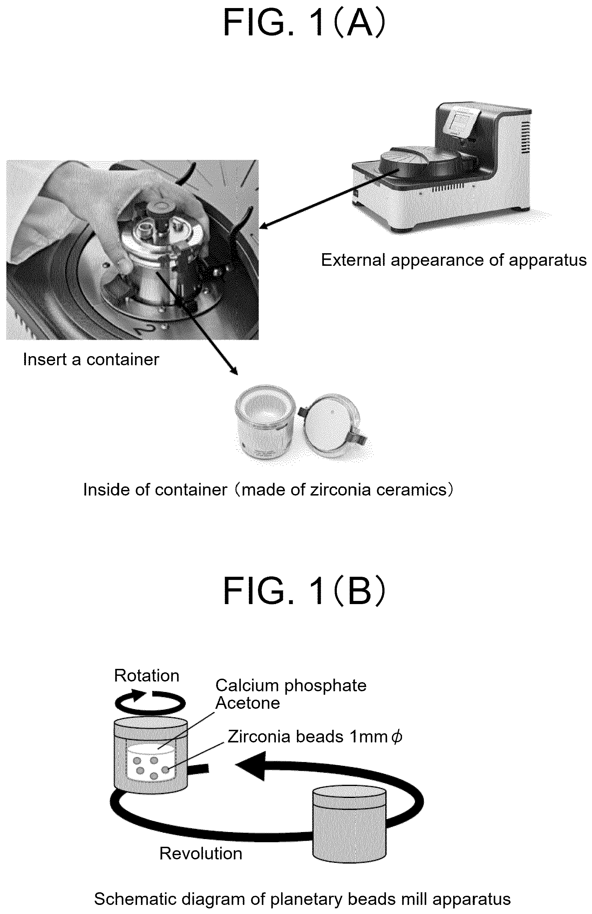

In the present invention, the term "bead mill apparatus" refers to a grinding machine used to apply a mechanochemical treatment to particles, and which uses beads with a diameter of 0.015 to 0.5 mm as grinding media (see Figure 1 (A)). In a planetary bead mill, the particles loaded into the vessel are subjected to repeated alternation of rotation and revolution, and the beads used as grinding media can apply strong impact energy to the particles, enabling them to be ground with a higher grinding efficiency (see Figure 1 (B)). There are different types and models of bead mill apparatus, however, as long as they are able to finely grind calcium phosphate particles using beads, they can be used as bead mill apparatus for the present invention.

### <Wet grinding>

In the present invention, "wet grinding" refers to a method of grinding by mixing a powder and a liquid (solvent) to form a slurry, and then adding balls or beads to the container and stirring the mixture. This is contrasted with dry grinding, which involves grinding without mixing the liquid (in air). In ball mill processing/bead mill processing, if the particles are ground in a dry state, the load is applied to the ground particles one after another, and they become clogged, so it is easy to form hard aggregates during the grinding process. However, even in wet grinding, if a solvent is used that dissociates H⁺ and has polarity, the ground particles will form aggregates through hydrogen bonding, so it is necessary to take measures to avoid aggregation of the ground particles.

### <Hydrogen bond>

In academic terms, a hydrogen bond is a non-covalent attractive interaction formed by a hydrogen atom bonded to an atom with a high electronegativity (a negative atom) via a covalent bond, and an isolated electron pair such as nitrogen, oxygen, sulfur, fluorine, or a pi electron system located nearby. In this invention, the term "hydrogen bond" refers to the phenomenon in which calcium phosphate particles that have been finely ground and amorphized by a bead mill and then bonded to H⁺ that has dissociated from the solvent used in wet grinding acquire an electrical negativity of δ - and, in that state, generate a weak electrical bond between particles by attracting the H⁺ bonded to adjacent calcium phosphate particles. When water is used as a solvent for wet grinding in a bead mill, because water is polar molecule that dissociates H⁺, the calcium phosphate particles ground in the bead mill are likely to form hydrogen bonds with each other, and as a result, the particles are likely to aggregate. Since acetone (C₃H₆O) is a non-polar molecule that does not dissociate H⁺, it does not form hydrogen bonds and thus can be preferably used in the wet grinding of this invention. When a large amount of water is present during grinding, the calcium phosphate that has been ground reacts with the water and changes into HAp, which is difficult to dissolve in the body. Thus, there is also the advantage that using acetone makes it possible to prevent this phenomenon.

### <Metal ions, semi-metal ions>

In the present invention, "metal ions" refers to ions separated from metal salt particles such as magnesium and silver by receiving impact energy of a bead mill to the powder of the metal salt particles. In a preferred embodiment of the invention, the metal ions separated from the metal salt particles recombine with calcium phosphate particles that have had their chemical potential increased by the impact energy of the bead mill. In the present invention, "semi-metallic ions" refers to semi-metallic ions separated from semi-metallic salt particles of semi-metals such as silicon and boron by receiving the impact energy of a bead mill. In a preferred embodiment of this invention, semi-metallic ions separated from semi-metallic salt particles recombine with calcium phosphate particles whose chemical potential has increased due to receiving the impact energy of a bead mill.

### Embodiment 1 (Amorphize β -TCP particles by bead milling)

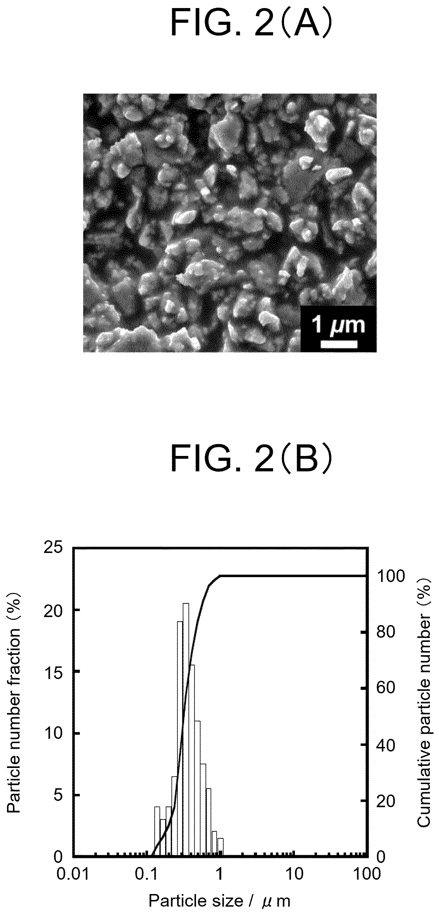

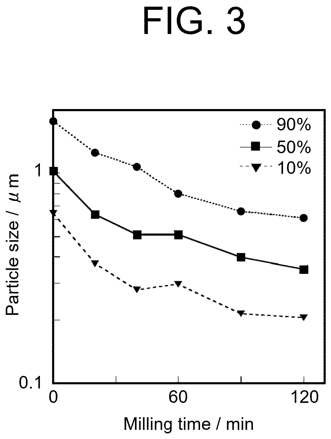

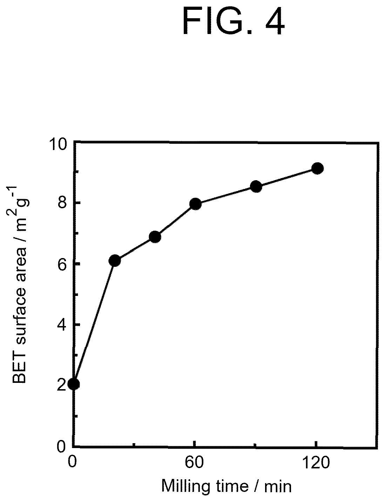

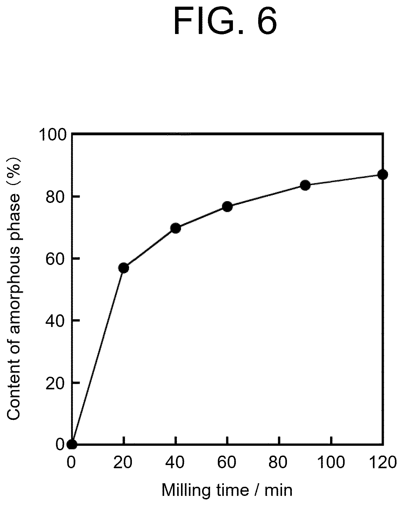

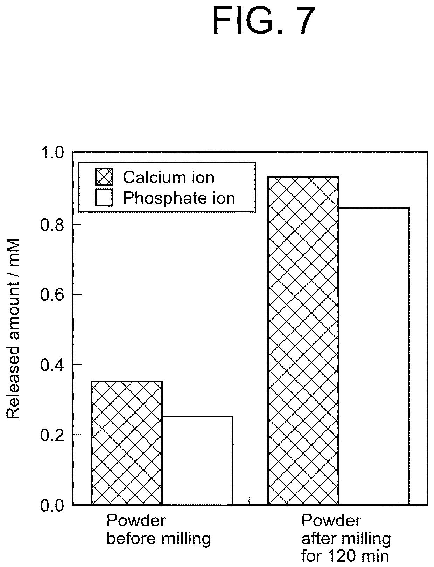

(1) A powder of *β* -TCP particles with a diameter of 1 to 5 *µ*m is put in a zirconia pot (volume capacity 30 to 60 m1) of a planetary bead mill device together with 35 to 50 g of beads with a diameter of 0.6 to 1.4 mm, in a quantity that the ratio of apparent volume of the *β* - TCP particles to beads is 1: 2 to 4 (if the media diameter of the beads is large, the space between the beads becomes large and the apparent volume becomes large, and conversely, if the media diameter of the beads is small, the space between the beads becomes small and the apparent volume becomes small). Then, add 15 to 20 m1 of acetone, and make the planetary ball mill apparatus rotate and spin at a predetermined speed.
(2) The *β* -TCP particles are ground by receiving impact of the beads, and their particle size is reduced to 0.1-1.2*µ*m (Figs. 2 and 3), while their specific surface area is increased 3-5 times (Fig. 4), and the crystal structure of the *β* -TCP particles is partially amorphized, reducing their crystallinity to 50-10% (Figs. 5 and 6). The powder of *β* -TCP particles that have been pulverized and made fine does not aggregate.
(3) When the powder of amorphized *β* -TCP particles is immersed in an aqueous solution (a tris-HCl buffer solution with a pH of 7.40), *β* - TCP is dissolved and calcium ions and phosphate ions are released from the particles (Figure 7).

### Embodiment 2 (β -TCP particles are amorphized to carry metal ions)

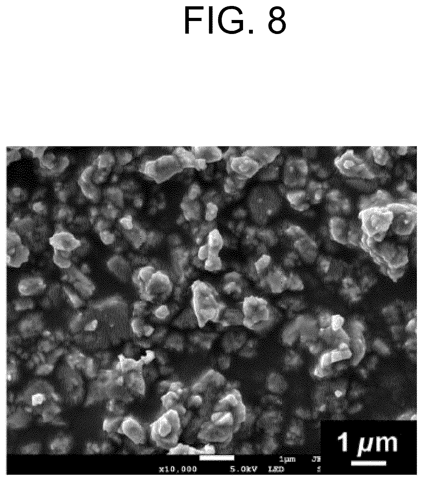

(1) A mixture of *β* -TCP powder and metal salt powder in a weight ratio of 0.99 to 0.99 to 0.01 to 0.1 is made, and the mixture of the powders is put in a zirconia vessel (volume capacity 30-60ml) of a planetary ball mill apparatus together with 15-20ml of acetone and 35-50g of beads with a diameter of 0.6-1.4mm, and the planetary ball mill apparatus is revolved and rotated at a predetermined speed.
(2) Upon receiving impact from the beads, the *β* -TCP particles are ground so that their particle size is decreased and specific surface area is increased, while the crystal structure of the *β* -TCP particles partially becomes amorphous. The metal salt particles that are fed into the zirconia vessel of the planetary ball mill are impacted by the beads, and the metal ions are separated from the particles. The separated metal ions coordinate with the phosphate ions of the amorphized *β* -TCP particles.
(3) After a high-speed rotation of the planetary ball mill is continued for a certain period of time and left still, the metal ions separated from the metal salt particles are immobilized in a state of coordination bonding with the phosphate ions of the amorphized *β* -TCP particles (Figs. 8 and 9). Thereafter, the metal salt particles are separated from the powder mixture by sieving, and the *β* -TCP particles containing the metal ions are collected.

The *β* -TCP particles whose crystal structure has been partially amorphized by bead milling have higher solubility in aqueous solutions and body fluids than *β* -TCP particles that have not undergone bead milling, and they dissolve in aqueous solutions and body fluids to release calcium and phosphate ions early on (Figure 7). In addition, the metal ions bound to the amorphized and ionized *β* -TCP particles are released together with the calcium and phosphate ions as the *β* - TCP particles dissolve (Figure 10).

The mechanism of grinding of particles by bead milling, amorphous phase formation, and metal/semimetal ion loading is not necessarily clear. According to the knowledge of the inventor of present invention, when beads (about 1 mm in diameter) are placed in the vessel of a bead mill apparatus and calcium phosphate (*β* -TCP) powder and metal salt particle powder are added and the apparatus is rotated at a high speed, the particles of the powder are subjected to a large energy due to intense mechanical impact, causing the bonds of the particles be broken or distorted. When the bonds of calcium phosphate particles are broken or distorted, their chemical potential (which becomes energy when multiplied by the amount of substance) becomes high. Once broken, the bonds cannot remain in that state (as ions) forever, depending on the surrounding conditions, so they try to recombine with nearby ions to lower their potential. When calcium phosphate particles are mixed with metal salt particles such as silver phosphate and magnesium oxide, or with semimetal salt particles such as silicates and boron compounds, same thing happens to these compounds. Metal or semimetal ions are separated from the metal salt or semimetal salt particles, and the separated metal or semimetal ions recombine with the calcium phosphate particles, which have high chemical potential. While the ball mill is rotating at high speed, the impact energy is constantly applied, so there is not enough time to create the original bonds with the lowest potential, and the situation continues where the particles cannot be neatly aligned. Even if the energy is a little high, it is lower than when they exist independently, so the arrangement of the ions becomes random, and when the grinding is finished, the energy from the outside stops being applied, and as a result, the ions cannot move, so they solidify almost as they are and become amorphous calcium phosphate. In the environment where the particles are being ground and mixed, calcium ions, silver ions, phosphate ions and borate ions coexist, and they form a randomly arranged (weaker than the original compound bond) bond state (amorphous).

### Embodiment 3 (Amorphize β -TCP particles and load metallic ions)

(1) Similarly to the method of embodiment 2, a mixture of *β* -TCP powder and semi-metallic salt powder in a weight ratio of 0.99-0.9 to 0.01-0.1 is added to a zirconia vessel of a planetary ball mill apparatus together with a predetermined amount of acetone and beads. The planetary ball mill is revolutionized and rotated at a constant speed, thereby the *β* -TCP particles are crushed to reduce their particle size and increase their specific surface area.
(2) Upon receiving impact from the beads, the *β* -TCP particles are partially amorphized and partially ionized, and the semi-metallic ions are separated from the semi-metallic salt particles, and the separated semi-metallic ions are coordinated with the phosphate ions of the *β* - TCP particles.
(3) After the high-speed rotation of the planetary ball mill apparatus is continued for a certain period of time, the semi-metallic ions separated from the semi-metallic salt particles are coordinate bonded to the phosphate ions of the amorphous *β* -TCP particles and immobilized. After the immobilization process, the semi-metallic salt particles are separated from the powder mixture by sieving and washing so that *β* -TCP particles containing semi-metallic ions are recovered.

The *β* -TCP particles whose crystal structure has been partially amorphized by bead milling have higher solubility in body fluids than the *β* -TCP particles that have not undergone bead milling, and they dissolve in body fluids to release calcium ions and phosphate ions. In addition, the semi-metallic ions bound to the amorphized and ionized *β* -TCP particles are released together with calcium ions and phosphate ions into the affected area of the body as the *β* -TCP particles dissolve, resulting in promotion of bone formation.

### Embodiment 4 (Amorphize HAp particles and load metallic ions or semi-metallic ions)

(1) Similarly to the method of embodiment 2, a mixture of HAp particle powder and metal salt particle powder or semi-metal salt particle powder is prepared in a weight ratio of 0.99-0.9 to 0.01-0.1, and then the powder mixture is put into a zirconia vessel of a planetary ball mill apparatus together with a predetermined amount of acetone and beads having a predetermined diameter. The planetary ball mill apparatus is made to rotate and revolve at a predetermined speed.
(2) Upon receiving impact from the beads, HAp particles are ground so that their particle size is decreased and specific surface area is increased, and the crystal structure of the HAp particles becomes partially amorphous and partially ionized. Metal ions or semi-metal ions are separated from the metal salt particles or semi-metal salt particles, and the separated ions coordinate with the phosphate ions of the HAp particles.
(3) After the high-speed rotation of the planetary ball mill apparatus is continued for a certain period of time, the amorphous HAp particles are immobilized in a state where the metal or semi-metal ions separated from the metal salt or semi-metal salt particles are coordinated to the phosphate ions of the HAp particles. After the immobilization process, the metal or semi-metal salt particles are separated from the mixture of the powder by sieving and washing, and HAp particles containing metal or semi-metal ions are recovered. By the processing of HAp in a bead mill, the amorphous part of HAp, which is not soluble in water, acquires solubility.

### Embodiment 5 (Load silver ions on amorphized β -TCP particles)

(1) Mix the powder of *β* -TCP particles and the powder of silver phosphate particles in a weight ratio of 0.99-0.9 to 0.01-0.1. Put the mixture of powders into a zirconia vessel of a planetary ball mill apparatus together with a predetermined amount of acetone and beads having a predetermined diameter. The planetary ball mill is revolved and rotated at a constant speed, thereby the *β* -TCP particles are ground such that the particle size is decreased and specific surface area of the particles is increased.
(2) The *β* -TCP particles are partially amorphized and partially ionized by the impact of the beads, and silver ions are separated from the silver phosphate particles, and the separated silver ions coordinate with the phosphate ions of the *β* -TCP particles.
(3) After the high-speed rotation of the planetary ball mill apparatus is continued for a certain period of time, the amorphous *β* -TCP particles are immobilized in a state in which the silver ions separated from the metal salt particles are coordinated to the phosphate ions. After the immobilization process, the silver salt particles are separated from the mixture of the above-mentioned powders by washing them through a sieve, and the *β* -TCP particles containing silver ions are recovered.

*β* -TCP particles whose crystal structure has been partially amorphized by bead milling have higher solubility in body fluids than *β* -TCP particles that have not been bead milled, and they dissolve in body fluids to release calcium ions and phosphate ions early. In addition, the silver ions bound to the amorphized and ionized *β* -TCP particles are released together with the calcium and phosphate ions into the affected part of the body as the *β* -TCP particles are dissolved, thereby antibacterial property is performed.

### Embodiment 6 (Inclusion of amorphized β -TCP particles in biodegradable fibers)

(1) *β* -TCP particles, amorphized *β* -TCP particles and PDLLA are mixed and stirred in acetone to prepare a particle dispersion. In a representative example, weight ratio of *β* -TCP particles, amorphized *β* -TCP particles, and PDLLA is 35%:35%:30%.
(2) The prepared particle dispersion is used as a spinning solution, and fibers with a diameter of 100-150 *µ*m are spun from the spinning solution using a wet spinning method. Because PDLLGA is amorphous and has low intrinsic viscosity, it is difficult to spin and form fibers using electrospinning method. However, PDLLGA is more easily broken down when in contact with body fluids than PLLGA, which is composed only of L-body, and is therefore suitable for early bone formation.

When a bone regeneration material made of PDLLGA resin fibers containing amorphized *β* -TCP particles is implanted in the affected area, the PDLLGA resin is dissolved early. The amorphized *β* -TCP particles contained in the PDLLGA resin fibers dissolve early when they come into contact with body fluids, and calcium ions and phosphate ions are released early into the affected area.

### Experiment 1

### <Outline of Experiment 1〉

(1) 40.5g of 1mm diameter beads and 13.5g (17ml) of acetone were placed in the zirconia vessel (45ml capacity) of the planetary bead mill. The beads and acetone were placed in the vessel up to 8/10 of the vessel capacity.
(2) Then, a quantity of *β* -TCP particles (Taihei Chemical Industry *β* - TCP-100, particle diameter 1-5*µ*m) was added to the mixture, in a ratio of 1:3 by apparent volume (the weight ratio of acetone to the total of *β* -TCP particles and beads was 1:7.5). The planetary ball mill apparatus was then rotated at a high speed, with a revolution at 400 rpm and a rotation at 800 rpm.
(3) The *β* -TCP particles (m-TCP particles) obtained were observed using a scanning electron microscope (SEM), and the particle size was measured (n=200). The specific surface area of the particles was measured using the nitrogen desorption method, and the structure was evaluated using XRD and TEM. In addition, m-TCP particles were immersed in a tris-HCl buffer solution (37° C, pH 7.40), and the ion releasing behavior and changes in the particles were investigated using ICP-AES and XRD.

### <Result of Experiment 1>

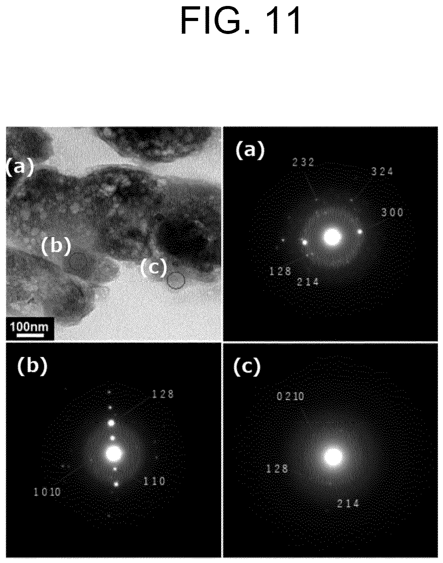

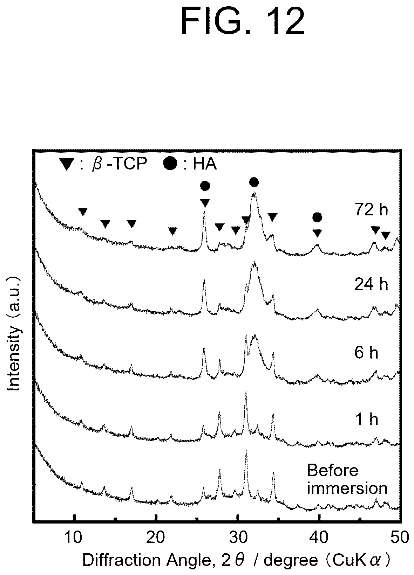

(i) Average diameter of the *β* -TCP particles was reduced to about 0.35 *µ*m by bead milling (Figure 3), and the specific surface area was increased by about 4.4 times (8.8 m2/g) (Figure 4). Due to the small media of beads (1 mm diameter) and high rotation speed, a large force was applied to the particles so that the particles were effectively ground in a short period of time of 120 minutes. After the processing, the beads were separated from the powder slurry by sieving, and the powder slurry was dried. When the powder was rubbed between the thumb and forefinger, no powder was felt on the fingertips.
(ii) In the XRD pattern of the beads-milled particles, a broad peak was observed along with a peak attributed to *β* -TCP (Fig. 5). In the electron diffraction image of these particles, a halo pattern was observed along with diffraction points attributed to *β* -TCP throughout the particles (Fig. 11).
(iii) In the tris-HCl buffer solution in which the bead-milled particles were immersed, there was a rapid increase in calcium and phosphate ions from the start of immersion to 24 h. In the XRD pattern of the particles after immersion, a new peak was observed that could be attributed to hydroxyapatite (HA) (Fig. 12). It is considered that the rapid increase in ion concentration increased the degree of supersaturation of the calcium phosphate phase, which resulted in precipitation HA.
(vi) This experiment showed that bead milling can amorphize a portion of *β* -TCP particles in a short period of time, and improve the solubility of the particles.

### Comparative experiment 1

Under the same condition as that of experiment 1, *β* -TCP particles were wet-milled using a planetary bead mill. However, the same amount of water was used as the solvent for wet-milling.

The result of above experiment showed that when the particles were crushed using water, they did not flow immediately when separated on a sieve, but rather it was observed that the crushed particles were tangled up in the field of beads. When the resulting powder slurry was dried and rubbed between the thumb and forefinger, it felt rough on the fingertips. According to inventor's experience, when something feels rough, it is roughly agglomerated to a diameter of 10 *µ*m or more.

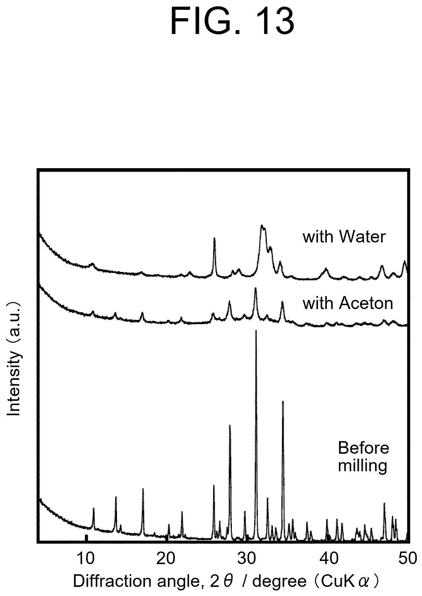

In addition, from the XRD of the particles ground with water, the *β* - TCP peak remained, and halo peaks due to the formation of the amorphous phase were observed at 25-40° . On the other hand, the particles crushed with water did not show the *β* -TCP peak, and only HA peak was observed. Existence of halo peaks could not be observed (Figure 13). It is considered that the calcium phosphate, which had become amorphous due to crushing, reacted with water to form apatite.

### Experiment 2

### <Outline of Experiment 2〉

(1) *β* -TCP powder and Ag₃PO₄ powder were mixed in a weight ratio of 99:1 or 95:5 so that 1.8g of powder of the mixture was prepared.
(2) 40.5g of beads having a diameter of 1mm and 13.5g (17ml) of acetone were put in a zirconia vessel (45ml capacity) of a planetary ball mill apparatus. After the beads and acetone were added to the vessel, the vessel was filled up to about 80% of its capacity. Then, the 1.8g of the mixed powder prepared above was added, and the ball mill apparatus was rotated at high speed with planetary revolution of 400rpm and rotation of 800rpm.
(3) When the planetary ball mill apparatus was rotated at high speed, the crystal structure of the *β* -TCP particles was partially amorphized due to the impact of the beads. Due to the impact of the beads, Ag ions were separated from the Ag₃PO₄ particles and the separated Ag ions (positive) coordinated with the phosphate ions (negative) of the *β* - TCP particles.
(4) After the high-speed rotation of the planetary ball mill apparatus described above continued for 2 hours, the amorphous *β* -TCP particles were immobilized in a state in which the Ag ions separated from the Ag₃PO₄ particles were coordinated to the phosphate ions.
(5) After the immobilization process described above, the Ag₃PO₄ particles were separated by sieving and washing out the mixture of the powder, and the *β* -TCP particles containing silver ions were recovered.

### <Result of Experiment 2>

(i) The beads used in the experiment had a diameter of 1 mm. By using beads having 1 mm in diameter, they could be roughly recovered by simply washing them with acetone through a sieve with an aperture of around 125 *µ*m. If beads having a diameter smaller than 1 mm were used, the effect of the milling would be increased, but a sieve with a smaller aperture would be required to prevent the beads from falling, so more time would be required for recovery.
(ii) After the powder of particles prepared by wet bead milling treatment of Ag₃PO₄ particles and *β* -TCP particles was immersed in a HEPES buffer solution (pH 7.40), ion concentration of the solution was measured using ICP-AES. Release of Ag ions was confirmed (Figure 10).

### Experiment 3

### <Outline of Experiment 3>

(1) *β* -TCP powder (Taihei Chemical Industry) and B₂O₃ powder (special grade, Xyda Chemical) was mixed in a weight ratio of 10-30 : 90-70. 1.8g of the mixture was prepared.
(2) 40.5g of beads having 1mm diameter and 13.5g (17ml) of acetone were put in a zirconia vessel (45ml capacity) of a planetary ball mill apparatus. The beads and acetone were put in the vessel so that they filled about 80% of the vessel. Then, 1.8g of the mixed powder prepared as described above was added, and the ball mill apparatus was rotated at high speed with revolution at 400rpm and rotation at 800rpm.
(3) When the planetary ball mill apparatus was rotated at high speed, crystal structure of the *β* -TCP particles was partially amorphized due to the impact from the beads. At the same time, the impact from the beads also caused some of the bonds of the B₂O₃ particles to become strained or broken, or new bonds to be formed, resulting in amorphization.
(4) After the high-speed rotation of the planetary ball mill apparatus described above was continued for 2 hours, the amorphous B₂O₃ particles were fused with the amorphous *β* -TCP phase by allowing the mixture to stand still.
(5) After the immobilization process described above, the treated material was separated by sieving and washing with acetone to recover the calcium phosphate particles containing boron ions.

### <Result of Experiment 3>

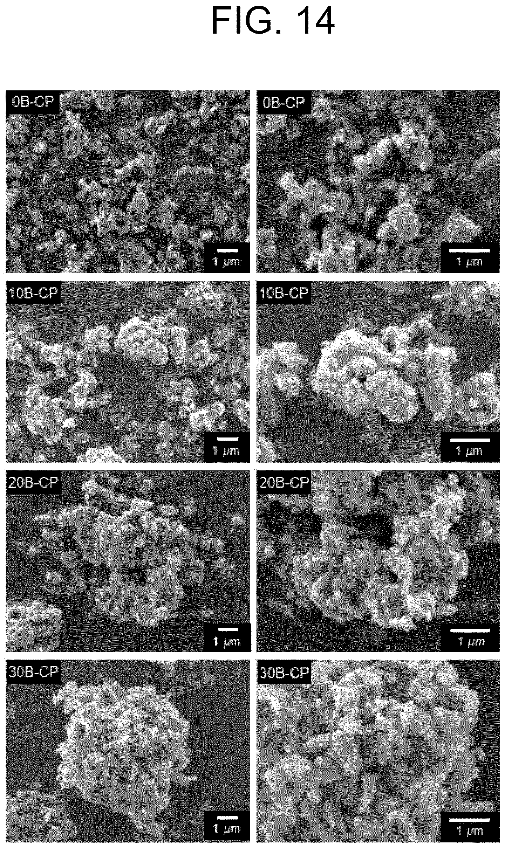

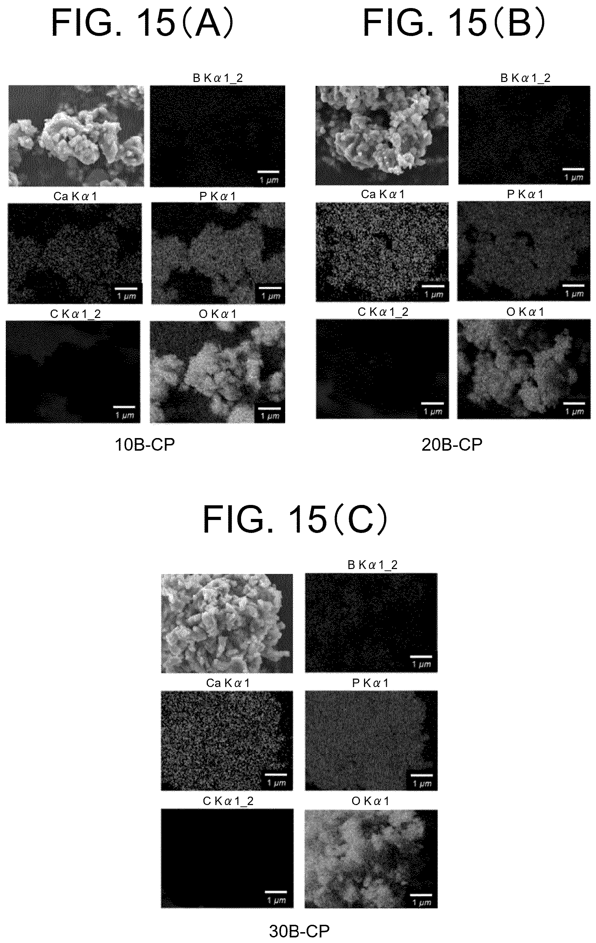

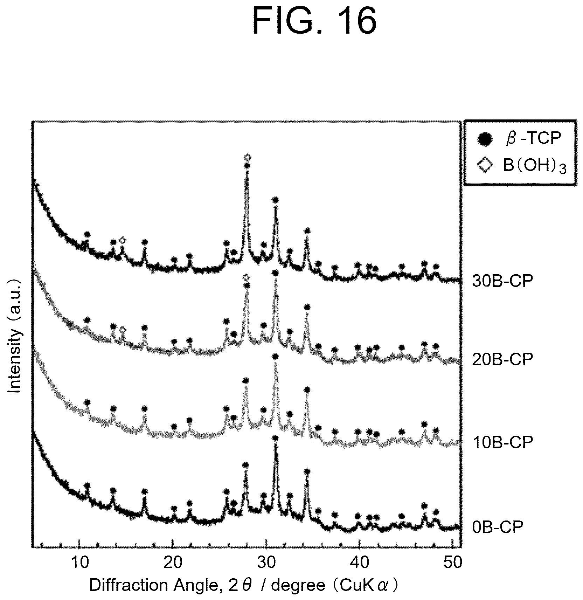

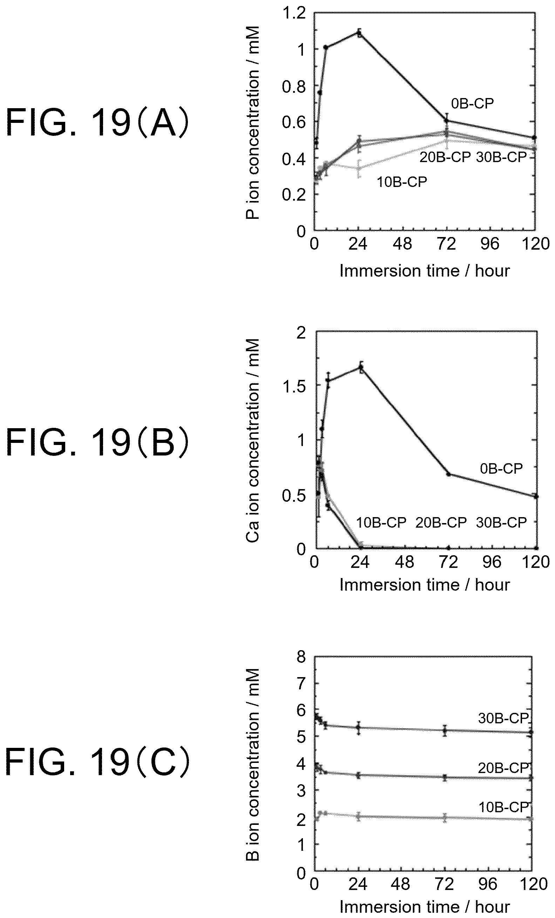

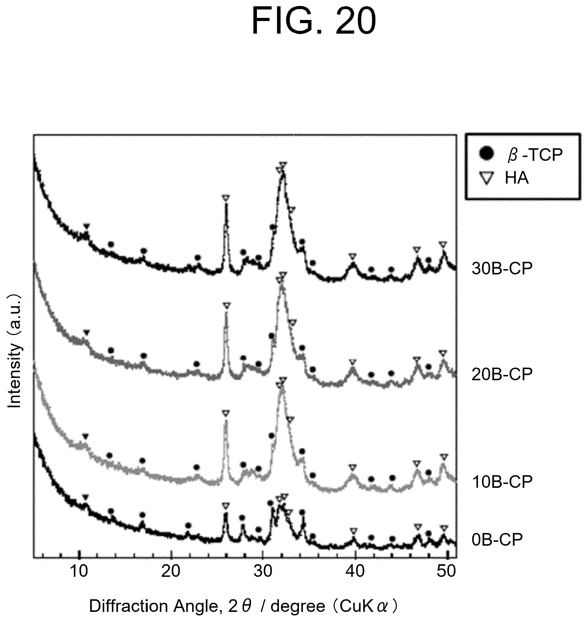

(i) The size of the particles obtained by grinding *β* -TCP powder and B₂O₃ powder together was approximately 0.1 *µ*m to 1 *µ*m, and the shape varied (Figure 14). In addition, the number of aggregated particles increased as the amount of boron added increased. It is considered that the aggregation occurred due to the reaction of boron with atmospheric water during the solid-liquid separation and drying processes, and it is condidered that the powder is highly reactive with water.
(ii) The element mapping of the EDS (Figure 15) showed that B, Ca, and P were distributed evenly. Although there are some accuracy issues with the low-energy position where boron (B_K*α*) is detected, it can be determined from the spectrum that it is present. When mapping, it was observed that it was distributed throughout the particles, and it existed in the same locations as calcium (Ca_K*α*) and phosphorus (P_K*α*). Therefore, it is considered that boron is dispersed and incorporated into the particles produced.
(iii) In the XRD pattern of the particles obtained (Figure 16), a peak (**●**) attributed to *β* -TCP and a halo peak due to the amorphous phase were confirmed. It is considered that the introduction of boron does not inhibit the amorphization of *β*-TCP by the mechanochemical treatment, and together with the results of Figure 3, it is considered that boron is also incorporated into the amorphous phase. In addition, when 20% and 30% B₂O₃ were mixed, peaks (◊) attributable to boric acid were observed. It is considred that boric acid was generated by the reaction of water in the air and boron oxide during the process after pulverization and during measurement.

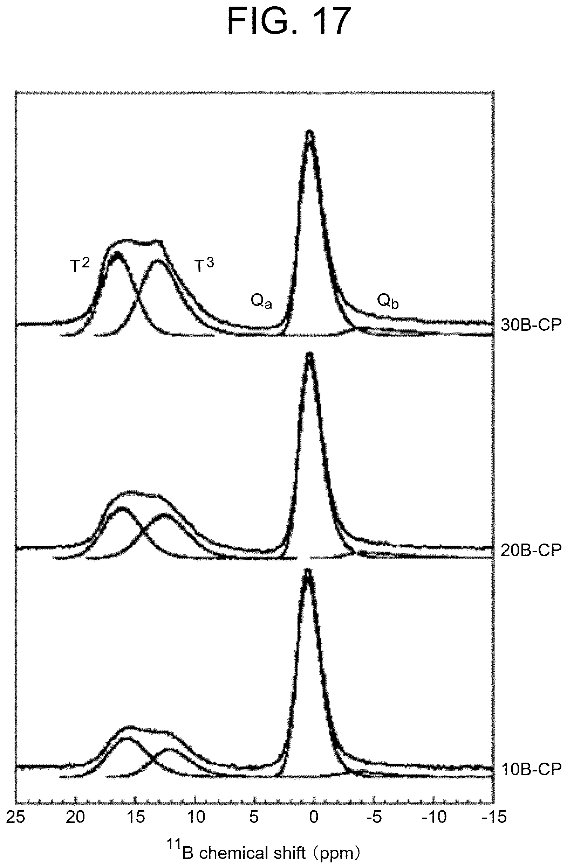

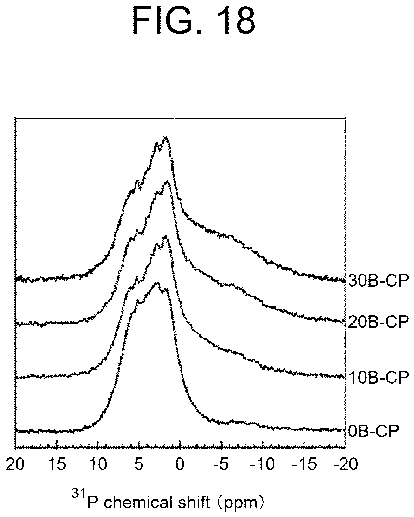

(iv) Figure 17 shows the 11B MAS-NMR spectrum of the particles obtained. It was shown that boron exists in T-structure (3-coordination of oxygen) and Q-structure (4-coordination of oxygen). Figure 18 shows the 31P MAS-NMR spectrum. With the addition of boron, a new peak related to the B-O-P bond was confirmed on the high-field side (around -5 ppm). In other words, through this mechanochemical treatment, boron is incorporated into the amorphous phase together with calcium phosphate to form a chemical bond.
(v) From the immersion test in tris-HCl buffer solution (Figure 19), the amount of borate ions eluted was greatly dependent on the amount of boron added. After 6 hours of immersion, there was no change in ion concentration, and all of the boron had eluted in the early stages of immersion. In the sample without adding boron, the concentration of calcium and phosphate ions eluted in the early stages decreased after 24 hours of immersion. This is considered to be due to the precipitation of HA, as seen in the XRD patterns (Fig. 20) after immersion. For the samples containing boron, the decrease in ion concentration was rapid, and the XRD patterns showed that the precipitation of HA was active. Borate glass (amorphous) has low chemical durability, and various ions are eluted due to the rapid decomposition of the B-O network structure in solution. This increases the concentration of calcium and phosphate ions, and also increases the pH, making it easier for HA to precipitate. The production of HA in a pseudo-physiological environment is related to the excellent bone-forming properties of the material, and the fact that the rate of this process can be increased shows the usefulness of adding B₂O₃ as a biomaterial.

### Experiment 4

### <Outline of Experiment 4〉

(1) A mixture of *β* -TCP powder (Taihei Chemical Industry) and amorphous silica powder (SYLISIA, Fuji Silysia) was prepared in a weight ratio of 95-90:5-10, and 1.8g of the mixture was prepared.
(2) 40.5g of beads with a diameter of 1mm and 13.5g (17ml) of acetone were put in a zirconia vessel (volume capacity 45ml) of a planetary ball mill apparatus. The beads and acetone were put in the vessel so that they were submerged to about 80% of the vessel's capacity. Then, 1.8g of the mixed powder prepared as described above was added, and the ball mill apparatus was rotated at high speed with a revolution of 450rpm and a rotation of 820rpm.
(3) When the planetary ball mill apparatus was rotated at a high speed, crystal structure of the *β* -TCP particles was partially amorphized due to the impact from the beads. The silica material is an amorphous powder prepared using the sol-gel method.
(4) After the high-speed rotation of the planetary ball mill apparatus described above was continued for 2 hours, the amorphous silica particles were fused with the amorphized *β* -TCP phase by allowing the mixture to stand still.
(5) After the immobilization process described above, the mixture was separated by sieving and washing with acetone, and the calcium phosphate particles containing silicate ions were recovered.

### <Result of Experiment 4>

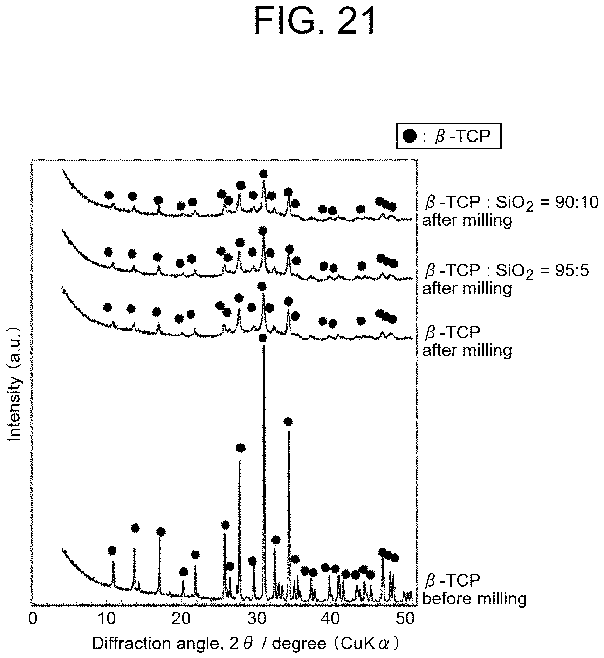

From the XRD pattern in Figure 21, it is observed that the amorphization of *β* -TCP progresses even when silica is mixed in. Because the silica mixed in is amorphous powder produced using the sol-gel method, it does not appear in the XRD pattern.

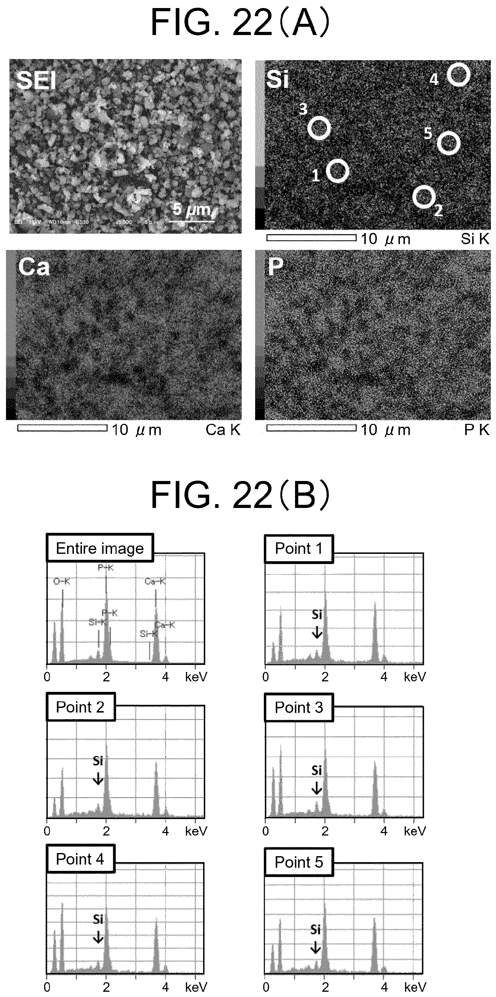

From the EDS mapping results in Figure 22, it is observed that silicon is present in approximately the same position as phosphorus and calcium, and that there are no significant differences in the spectra for each of the points 1 to 5, indicating that it is distributed throughout.

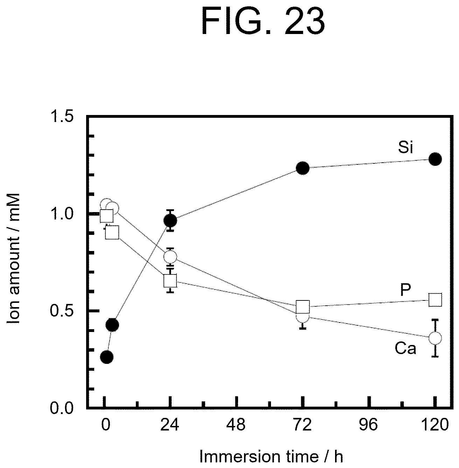

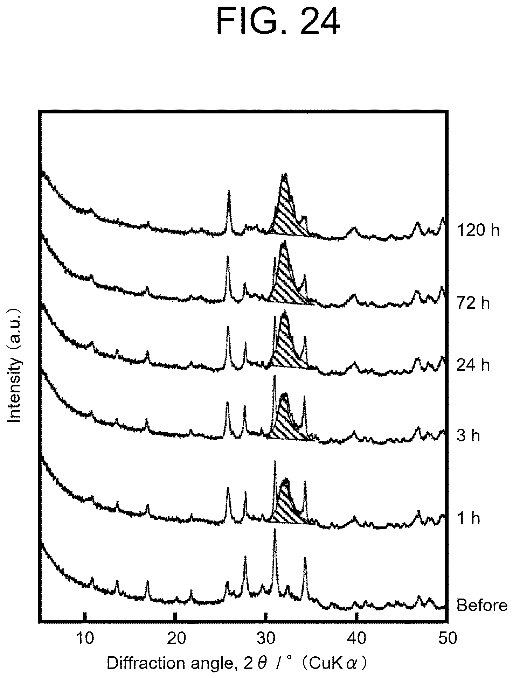

When the ion content was examined after 50 mg of particles mixed with 5% silica were immersed in 20 ml of a tris-HCl buffer solution (37° C, pH 7.40) (Figure 23), calcium and phosphate ions rapidly increased after immersion and then gradually decreased. From the XRD pattern in Figure 24, it was found that HA was being produced. Therefore, the calcium and phosphate ions decreased because they were consumed. On the other hand, it was found that silicon was gradually released over time, but after 24 hours the amount of release appeared to gradually decrease. This is considered that because it is partially incorporated into the HA formed. Since silicate ions are known to have an effect of promoting bone formation, this type of release behavior is desirable.

### Experiment 5

### <Outline of Experiment 5〉

The beads mill-processed calcium phosphate particle powder (m-TCP) prepared in Experiment 1 and the poly (D,L-lactic acid-gluconic acid) copolymer (PDLLA) pellets were mixed in a weight ratio of 70:30, and the mixture was dispersed by adding it to an acetone solution and stirring it. The ratio of powder to acetone was 1:6.

The prepared spinning solution was filled into the syringe of the wet spinning apparatus, and by pushing the filled spinning solution out of the nozzle, a biodegradable fiber was produced that contained 30% PDLLA and 70% calcium phosphate particles (Fiber A).

In addition, fibers with a weight ratio of 35:35:30 for *β* -TCP:m-TCP:PDLLA (Fiber B) and fibers with a weight ratio of 70:30 for *β* - TCP:PDLLA (Fiber C) without m-TCP were produced using the same method as above for comparison. Provided, however that, the ratio of powder to acetone was 1:5.

### <Result of Experiment 5>

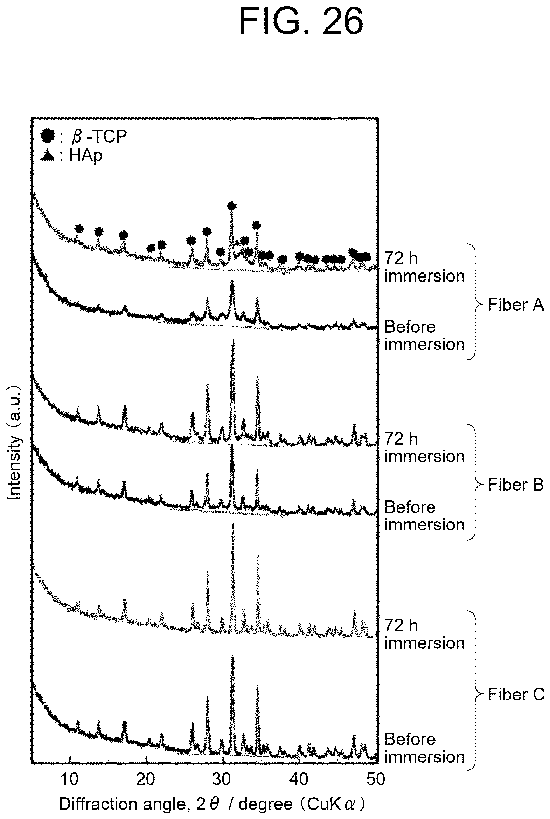

(i) Since the m-TCP particle powder was not aggregated, it was easy to prepare a spinning solution in which the particles were dispersed in the liquid by adding the powder to the solvent together with the resin and stirring it.
(ii) On the other hand, in order to produce a fiber containing both *β* -TCP and m-TCP (Fiber B), it was necessary to prevent the larger-particle *β* -TCP from settling as much as possible due to the difference in particle size. Because of that, a fiber with relatively well-dispersed particles was obtained by preparing a spinning solution with higher viscosity. Similarly, when only *β* -TCP was dispersed (Fiber C), it was possible to obtain fibers with relatively well-dispersed particles by making a spinning solution with higher viscosity (ratio of powder amount to acetone: 1:5),
(iii) When the ion content was examined after 20mg of each of Fibers A, B and C were immersed in 20ml of a tris-HCl buffer solution (37°C, pH7.40) (Figure 25), after 6 h immersion, Fiber A and Fiber B released 6 to 7 times more calcium ions and phosphate ions than Fiber C. This is considred to be due to the dissolution of the amorphous phase in m-TCP. The amount of each ion decreased after 72 h immersion in Fiber A. From the XRD, it was confirmed that HA was being produced (Fig. 26). Therefore, it can be said that the amount decreased because calcium ions and phosphate ions were consumed.
From these results, it is clear that using m-TCP as a biodegradable fiber gives the ability to actively release calcium ions and phosphate ions.

While present invention has been explained by referring to the embodiments of the present invention, scope of the present invention is not limited to these embodiments, and should be limited only by recitation of the claims of this application.

## Claims

1. A method of modifying crystalline calcium phosphate particles into amorphous calcium phosphate by wet-grinding using a bead mill apparatus, comprising:
putting a powder of crystalline calcium phosphate particles having a diameter of 1-5 *µ*m and beads having a media diameter of 0.015-2.0 mm in a vessel of a bead mill apparatus such that ratio of the apparent volume of the powder to the beads becomes 1:2-4, and
adding acetone to the mixture of crystalline calcium phosphate powder and beads in the vessel such that weight ratio of the acetone to the mixture is 5-10 : 1, and stirring the mixture so that a slurry containing the calcium phosphate particles and beads in the acetone is prepared, and
rotating the bead mill apparatus at a predetermined rotation rate per minute for a predetermined time, thereby grinding the powder of the crystalline calcium phosphate particles contained in the slurry to a particle size of 0.1-1.2 *µ*m without forming aggregates so that the crystalline calcium phosphate particles are partially amorphized by receiving impact from the beads and crystallinity of the calcium phosphate particles is reduced to 50-10%.

2. The method of claim 1, wherein the bead mill apparatus is a planetary type having a function of rotation and revolution.

3. The method of claim 1 or 2, wherein the crystalline calcium phosphate particles are *β* -TCP or HAp.

4. The method of claim 1 or 2, wherein the metal salt is silver phosphate.

5. The method of claim 1 or 2, wherein the semi-metal salt is a silicate or a borate.

6. The method of claim 1 or 2, wherein the powder of the crystalline calcium phosphate particles and the powder of the metal salt particles or the semi-metal salt particles are mixed in a weight ratio of 0.99-0.9 to 0.01-0.1, and the mixture of the powders is put into the vessel of the planetary bead mill apparatus together with a predetermined amount of acetone and beads, and the planetary bead mill apparatus is rotated at a predetermined rate per minute, thereby causing the metal ions or semi-metal ions separated from the metal salt particles or semi-metal salt particles so that the separated metal or semi-metal ions are coordinated to the phosphate ions of the amorphized calcium phosphate particles.

7. Calcium phosphate particles carrying metal or semi-metal ions in a gradually releasable manner, wherein
crystalline structure of the calcium phosphate particles is partially modified to an amorphous state by subjecting the particles to a bead milling,
metal or semi-metal ions are coordinated to the phosphate ions of amorphous part of the calcium phosphate particles so that the metal or semi-metal ions are carried on the calcium phosphate particles in a manner that the metal or semi-metal ions can be gradually released from the particles,
when the calcium phosphate particles carrying the metal or semi-metal ions are implanted in a body and come into contact with body fluids, the amorphous calcium phosphate particles are dissolved and the metal or semi-metal ions are gradually released together with calcium ions and phosphate ions.

8. The calcium phosphate particles of claim 7, wherein a planetary-type apparatus having a function of rotation and revolution is used for the bead milling.

9. The calcium phosphate particles of claim 7 or 8, wherein the crystalline calcium phosphate particles are *β* -TCP or HAp.

10. The calcium phosphate particles of claim 7 or 8, wherein the metal salt is silver phosphate.

11. The calcium phosphate particles of claim 7 or 8, wherein the semi-metallic salt is a silicate or a borate.

12. A method of producing a bone regeneration material comprising biodegradable fibers containing calcium phosphate particles that are modified to an amorphous state, the method comprising:
putting a powder of crystalline calcium phosphate particles having a diameter of 1- 5 µm and beads having a media diameter of 0.015-2.0 mm in a vessel of a bead mill apparatus such that ratio of apparent volume of the powder to the beads becomes 1:2-4,
adding acetone to the mixture of crystalline calcium phosphate powder and beads in the vessel such that weight ratio of the acetone to the beads is 5-10 to 1, and stirring the mixture so that a slurry containing the calcium phosphate particles and beads in the acetone is prepared,
rotating the bead mill apparatus at a predetermined rotation rate per minute for a predetermined time, thereby grinding the powder of the crystalline calcium phosphate particles contained in the slurry to a particle size of 0.1-1.2 µm without forming aggregates so that the crystalline calcium phosphate particles are partially amorphized by receiving impact from the beads, and crystallinity of the calcium phosphate particles is reduced to 50-10%,
adding the powder of the amorphized calcium phosphate particles to a solvent together with biodegradable resin and stirring to produce a spinning solution in which the amorphized calcium phosphate particles are dispersed,
filling the spinning solution into a syringe of a spinning apparatus and extruding the spinning solution from a nozzle to spin biodegradable fibers, and
depositing the biodegradable fibers excluded from the nozzle in a collector and collecting the biodegradable fibers from the collector.

13. The method of claim 12, wherein bead mill apparatus is a planetary-type apparatus having a function of rotation and revolution.

14. The method of claim 12 or 13, wherein the crystalline calcium phosphate particles are *β* -TCP or HAp.

15. The method of claim 12 or 13, wherein metal or semi-metal ions are bound to calcium phosphate particles that are modified to amorphous.

16. A bone regeneration material comprising a biodegradable fiber containing calcium phosphate particles that are modified to amorphous state, the bone regeneration material is produced by the method comprising:
putting a powder of crystalline calcium phosphate particles having a diameter of 1-5 µm and beads having a media diameter of 0.015-2.0 mm in a vessel of a bead mill apparatus such that ratio of apparent volume of the powder to the beads becomes 1:2-4,
adding acetone to the mixture of crystalline calcium phosphate powder and beads in the vessel such that weight ratio of the acetone to the mixture is 5-10 to 1, and stirring the mixture so that a slurry containing the calcium phosphate particles and beads in the acetone is prepared,
rotating the bead mill apparatus at a predetermined rotation rate per minute for a predetermined time, thereby grinding the powder of the crystalline calcium phosphate particles contained in the slurry to a particle size of 0.1-1.2 µm without forming aggregates so that the crystalline calcium phosphate particles are partially amorphized by receiving impact from the beads, and crystallinity of the calcium phosphate particles is reduced to 50- 10%,
adding the powder of the amorphized calcium phosphate particles to a solvent together with biodegradable resin and stirring to produce a spinning solution in which the amorphized calcium phosphate particles are dispersed,
filling the spinning solution into a syringe of a spinning apparatus and extruding the spinning solution from a nozzle to spin biodegradable fibers,
depositing the biodegradable fibers excluded from the nozzle in a collector and collecting the biodegradable fibers from the collector.

17. The method of claim 16, wherein the bead mill apparatus is a planetary-type apparatus having a function for rotation and revolution.

18. The bone regeneration material of claim 16 or 17, wherein the calcium phosphate particles modified to the amorphous state carries metal or semi-metal ions that can be released gradually.
